# EUROPEAN PATENT SPECIFICATION

(11) **EP 2 276 504 B1**
(45) Date of publication and mention of the grant of the patent: **02.11.2011**
(21) Application number: 09750681.0
(22) Date of filing: 19.05.2009
(51) Int. Cl.: A61K 38/39, A61K 35/60, A61K 9/16, A61P 9/10

(54) **Medicament and formulations thereof for use in reducing or preventing atherosclerosis**
Medikament und Formulierung dieses enhaltend zur Verringerung oder Verhinderung von Atherosklerose
Médicament et des formulations de celle-ci pour une utilisation dans la réduction ou la prévention de l'athérosclérose

(30) Priority: 20.05.2008 JP 2008131621
(43) Date of publication of application: 26.01.2011
(73) Proprietor: Ishii, Hikaru, Tokyo 103-0024 (JP)
(72) Inventor: Ishii, Hikaru, Tokyo 103-0024 (JP)
(74) Representative: Delorme, Nicolas
(86) International application number: PCT/JP2009/059490
(87) International publication number: WO 2009/142319

(56) References cited:
- DATABASE WPI Week 200645 Thomson Scientific, London, GB; AN 2006-439080 XP002539426 -& JP 2006 166807 A (ISHIKAWA N) 29 June 2006 (2006-06-29)
- KIM S-K ET AL: "Isolation and Characterization of Antioxidative Peptides from Gelatin Hydrolysate of Alaska Pollack Skin" JOURNAL OF AGRICULTURAL AND FOOD CHEMISTRY, AMERICAN CHEMICAL SOCIETY, WASHINGTON, US, vol. 49, 1 January 2001 (2001-01-01), pages 1984-1989, XP002979282 ISSN: 0021-8561
- LI ET AL: "Isolation and identification of antioxidative peptides from porcine collagen hydrolysate by consecutive chromatography and electrospray ionization-mass spectrometry" FOOD CHEMISTRY, ELSEVIER SCIENCE PUBLISHERS LTD, GB, vol. 102, no. 4, 31 January 2007 (2007-01-31), pages 1135-1143, XP005892980 ISSN: 0308-8146
- TAFFIN, A AND PLUVINET R: "Hydrolyzed collagen" WELLNESS FOOOD EUROP, [Online] vol. 14, November 2006 (2006-11), pages 1-5, XP002539547 Internet Retrieved from the Internet: URL:http://www.harnisch.com/well/files/pdf /306/hydrolyzed_collagen.pdf>
- JUN S-Y ET AL: "Purification and characterization of an antioxidative peptide from enzymatic hydrolysate of yellowfin sole (Limanda aspera) frame protein" EUROPEAN FOOD RESEARCH AND TECHNOLOGY, SPRINGER VERLAG, HEIDELBERG, DE, vol. 219, 1 January 2004 (2004-01-01), pages 20-26, XP002979283 ISSN: 1438-2377
- DONG ET AL: "Antioxidant and biochemical properties of protein hydrolysates prepared from Silver carp (Hypophthalmichthys molitrix)" FOOD CHEMISTRY, ELSEVIER SCIENCE PUBLISHERS LTD, GB, vol. 107, no. 4, 28 November 2007 (2007-11-28), pages 1485-1493, XP022368955 ISSN: 0308-8146
- NAGAI T SUZUKI N: "Isolation of collagen from fish waste material - skin, bone and fins" FOOD CHEMISTRY, ELSEVIER SCIENCE PUBLISHERS LTD, GB, vol. 68, 1 January 2000 (2000-01-01), pages 277-281, XP002956948 ISSN: 0308-8146
- "Fish Collagen - Mahura Nichiro Foods, Inc." 2008, pages 1-2, XP002539416 INTERNET Retrieved from the Internet: URL:http://www.food.maruha-nichiro.co.jp/e nglish/ffcd/product/pro00700.html>
- DATABASE BIOSIS [Online] BIOSCIENCES INFORMATION SERVICE, PHILADELPHIA, PA, US; December 2008 (2008-12), LAI CHIH-HSING ET AL: "Studies on antioxidative activities of hydrolysates from fish scales collagen of tilapia" XP002539419 Database accession no. PREV200800308889 & JOURNAL OF TAIWAN FISHERIES RESEARCH, vol. 15, no. 2, December 2008 (2008-12), pages 99-108, ISSN: 1018-7324
- ZHUANG YONGLIANG ET AL: "The scavenging of free radical and oxygen species activities and hydration capacity of collagen hydrolysates from walleye pollock (Theragra chalcogramma) skin" JOURNAL OF OCEAN UNIVERSITY OF CHINA, vol. 8, no. 2, June 2009 (2009-06), pages 171-176, XP002539417 ISSN: 1672-5182
- XINRONG PEI ET AL.,: "Marine collagen peptide isolated from Chum Salmon (Oncorhynchus keta) skin facilitates learning and memory in aged C57BL/6J mice"[Online] pages 1-8, XP002539418 INTERNET Retrieved from the Internet: URL:doi:10.1016/j.foodchem.2009.04.120>

## Description

### TECHNICAL FIELD

The present invention relates to a medicine for use in reducing or preventing atherosclerosis, and relates in particular to a formulation comprising collagen from fish skin for use in reducing or preventing atherosclerosis comprising said medecine that exhibit a high rate of absorption.

### BACKGROUND ART

Metabolic capabilities tend to deteriorate with age, leading to increased fatigue and reduced anatomical functions. Accordingly, in recent years, various tests have been conducted on the transdermal absorption and oral administration of antioxidants (such as vitamins), various hormone preparations, and unsaturated fatty acids as potential anti-aging treatments. However, these tests represent only one step in anti-aging treatment.

On the other hand, collagen represents 1/3 of biological proteins, and is the main structural protein in biostructures. Furthermore, it is now clear that collagen not only performs a simple mechanical function as a support structure for biological bodies, but also has extremely important biological roles in protecting cells and as an intercellular factor.

Accordingly, JP 7-278012 A proposes a metabolic accelerator that comprises a collagen protein or a hydrolysis product thereof as an essential component.

### SUMMARY

Upon aging, as the cells of blood vessel walls age, the blood vessels tend to lose elasticity, and blood platelets and cholesterol accumulate on the blood vessel walls, increasing the possibility of atherosclerosis.

The present invention provides a medicin for use in reducing or preventing atherosclerosis and a formulation comprising said medicine that suppress blood vessel aging and inhibit a atherosclerosis.

The medicine for use in reducing or preventing atherosclerosis and formulation of the present invention have the features described below.
(1) According to the present invention, the medicine for use in reducing or preventing atherosclerosis comprises a low molecular weight collagen derived from fish skin as an essential component, and further comprising minerals such as calcium and phosphorus, and any of the various vitamins.
   As described below, low molecular weight collagen derived from fish skin exhibits a higher rate of absorption than collagen obtained from other raw materials. Accordingly, the favorably absorbed amino acid peptides are reassembled, and the resulting in-vivo collagen promotes metabolism of the blood vessel walls. This causes endothelial cells from the blood vessel walls to peel away, meaning blood platelets and cholesterol adhered to the inner membrane are also gradually removed, thereby reducing the amount of fibrous plaques. As a result, the blood vessel walls are renewed in a substantially constant manner, and the adhesion of blood platelets and cholesterol can be inhibited. By regular administration of a medicine containing a low molecular weight collagen derived from fish skin as an essential component, the elasticity of the entire blood vessel wall can be improved, and the adhesion of blood platelets and cholesterol can be prevented.
(2) According to the present invention there is provided the medicine disclosed in (1) above, wherein the weight average molecular weight of the low molecular weight collagen derived from fish skin is approximately 3,000.
   Compared with a higher molecular weight collagen, the low molecular weight collagen described above is decomposed into amino acid peptides of even lower molecular weight by in-vivo enzymatic decomposition, and therefore the absorption rate within the body, for example via intestinal absorption, is very high, meaning the efficiency of the blood vessel wall regeneration process can be enhanced.
(3) According to another aspect of the present invention there is provided a formulation in which the medicine disclosed in (1) or (2) above is in a granular form.
   Compared with capsules or tablets, producing the formulation in granular form accelerates the enzymatic decomposition that occurs when the formulation reaches the intestine.

### BRIEF DESCRIPTION OF THE DRAWINGS

Embodiment(s) of the present invention will be described in detail based on the following figures, wherein:
FIG. 1 is a graph describing the relationship between the absorption of fish-derived collagen peptides and animal skin-derived collagen peptides;
FIG. 2 is a diagram illustrating the results of measuring the weight average molecular weight of one example of a collagen derived from fish skin that represents the main component of an anti-aging formulation according to an embodiment of the present invention; and
FIG. 3 is a schematic cross-sectional view of a blood vessel illustrating an example of arterial sclerosis.

### DESCRIPTION OF EMBODIMENTS

One example of a medicine and formulation of the present invention is described below with reference to the drawings.

Depending on the raw material, collagen can be broadly classified as either animal skin-derived collagen (such as pig skin-derived collagen and chicken skin-derived collagen) and fish-derived collagen. It is well known that animal skin-derived collagen has a higher allergenicity than fish-derived collagen, and therefore fish-derived collagen offers superior safety. Furthermore, fish-derived collagen includes collagen derived from fish skin and collagen derived from fish scales. Scale-derived collagen is obtained by chemical treatment of the hard fish scales using a strong hydrochloric acid, and therefore not only is there a possibility of the collagen containing residual hydrochloric acid, but because the hydrochloric acid randomly cleaves bioactive amino acid peptide linkages, there is a possibility that a portion of the resulting collagen peptides may have lost its bioactivity. In contrast, fish skin-derived collagen can be decomposed with no loss of the amino acid peptide linkage bioactivity by appropriate application of heat and selection of a specific enzyme, and therefore it is not only safe, but also enables the extraction of collagen peptides that exhibit superior bioactivity to those obtained from scale-derived collagen.

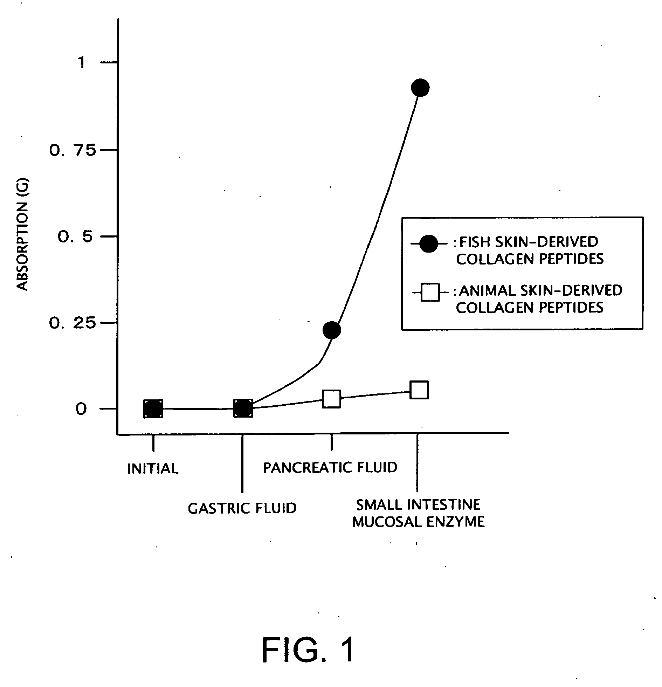

Furthermore, collagen derived from animal skin generally has an amino acid sequence that is peculiar to mammals, and tends to contain a larger amount of the collagen-specific amino acid known as proline than fish-derived collagen. Proline bonds to a hydroxyl group and exists in the form of hydroxyproline within collagen, and has a feature of exhibiting more powerful bonding than other amino acids. Because animal skin-derived collagen contains a much higher amount of hydroxyproline than that found in fish, it tends to be more difficult to decompose. It could be argued that this observation reflects the fact that many mammals are land-based animals, and that the necessity to protect their bodies from the very changeable weather and from foreign bodies resulted in the survival of those mammals for which the skin had been embryologically toughened. Accordingly, as can be seen in FIG. 1, even if an animal-derived collagen and a fish-derived collagen have the same molecular weight, the fish-derived collagen undergoes a much higher degree of decomposition than the animal-derived collagen. In other words, fish-derived collagen exhibits an extremely high in-vivo absorption rate (see FOOD Style 21, 2003.2, pp. 85 to 88).

However, in-vivo collagen is usually generated in three stages known as premature cross-linking, mature cross-linking and aged cross-linking. In this description, "premature cross-linking" refers to a state of intramolecular and intermolecular collagen cross-linking which exists in a high proportion in the collagen fibers of fetuses or newly born animals and which is readily dissolved by acid, "mature cross-linking" refers to cross-linking which is stable in the presence of acid or heat, is not reduced by sodium borohydride, and includes cross-linked structures such as pyridinoline or deoxypyridinoline, and "aged cross-linking" refers to a state in which cross-linking has progressed further via the Maillard reaction (also known as a glycation reaction) and under the influence of in-vivo active enzymes (see "The Collagen Story", by Daisaburo FUJIMOTO, published by Tokyo Kagaku Dozin Co., Ltd., 3rd edition, July 3, 2006, pp. 73 to 100) .

Generally, as aging proceeds, the proportion of aged cross-linking within the collagen that constitutes blood vessel walls tends to increase. As a result, the degree of collagen cross-linking in the blood vessel walls increases, which causes the blood vessel walls to harden, causes a loss in elasticity of the blood vessels themselves, and increases the accumulation of blood platelets and cholesterol on the blood vessel walls, making arterial sclerosis more likely.

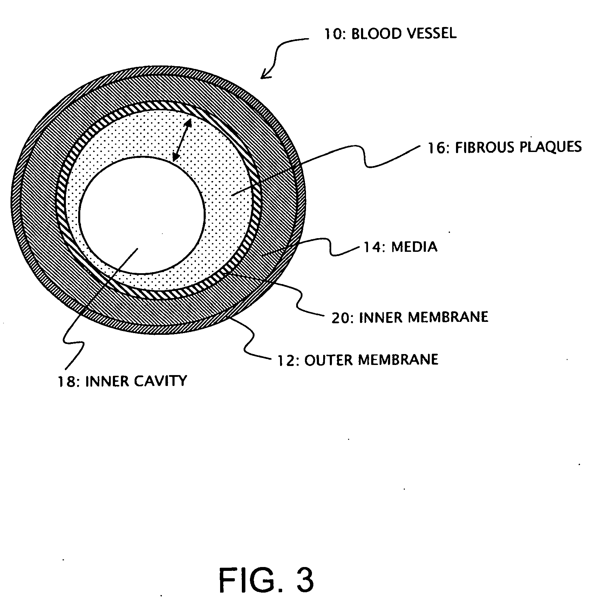

For example, FIG. 3 schematically illustrates a state in which arterial sclerosis has caused thickening and hardening of an artery wall. As can be seen in FIG. 3, in an artery wall suffering from atherosclerosis, the smooth muscle cells in the vicinity of an inner membrane 20 that exists on the inside of an outer membrane 12 and media 14 of a blood vessel 10 undergo repeated division, and the resulting monocytes permeate and are accumulated within the endothelial cells, and both the monocytes and the smooth muscle cells then start phagocytosis of fats contained within the blood, mainly cholesterol, causing the endothelial cells to also start accumulating lipids. As a result, the endothelial cells are enlarged, and when the endothelial cell layer cracks, blood platelets begin to bond to the exposed collagen fibers, resulting in the generation and accumulation of fibrous plaques 16 which are fatty structures that protrude into an inner cavity 18 of the blood vessel 10. The thickness of these accumulated fibrous plaques 16 is indicated by an arrow in FIG. 3. Generally, atherosclerosis is treated by using a vascular catheter to perform a balloon angioplasty and position a stent within the blood vessel. However, preventing occurrence of the above type of atherosclerosis is desirable. Current medications act by reducing vascular resistance to achieve an antihypertensive effect, or by reducing blood lipids to ameliorate hyperlipidemia, but no current medications can be claimed to act directly upon the blood vessel to provide a mechanism that achieves restoration and regeneration of the blood vessel.

Accordingly, the medicine of the present invention includes low molecular weight collagen derived from fish skin as an essential component.

As described above, low molecular weight collagen derived from fish skin exhibits a higher rate of absorption than collagen obtained from other raw materials, and therefore the favorably absorbed amino acid peptides are reassembled, and the resulting in-vivo collagen promotes metabolism of the blood vessel walls. This accelerates the metabolism of the endothelial cells on the inner cavity 18 side of the media 14 of the type of blood vessel 10 shown in FIG. 3, and the resulting turnover of the cells on the inner cavity 18 side of the inner membrane causes these cells to peel away, meaning the fibrous plaques 16 formed on the inner membrane are also removed and flushed into the bloodstream, where they can be treated as waste products and expelled from the body.

Moreover, by orally administering the vascular aging inhibitor of the present embodiment over a period of time, metabolism of the cells of the blood vessel wall can be accelerated, resulting in an improvement in the elasticity of the entire blood vessel wall and a dramatic suppression of the generation of fibrous plaques 16.

In addition, in the medicine according to the present invention, the weight average molecular weight of the aforementioned low molecular weight collagen derived from fish skin is approximately 3,000. Accordingly, compared with higher molecular weight collagen, the collagen is decomposed into amino acid peptides of even lower molecular weight by in-vivo enzymatic decomposition, and therefore the absorption rate within the body, for example via intestinal absorption, is very high, meaning the efficiency of the blood vessel wall regeneration process can be enhanced.

Examples of fish from which the fish-derived collagen can be obtained include bonito, tuna, marlin, cod, horse mackerel, mackerel, salmon, trout, saury, eel, tilapia, thread-sail filefish, grouper, halibut, flounder, sole, herring, sardine, sharks, rays, blowfish, yellow tail, scorpion fish, rockfish and pollock, and use of fish skin collagen from one of these fish is preferred.

Examples of the protein hydrolyzing enzyme that can be used for the collagen decomposition include neutral protease, alkaline protease, acidic protease and pepsin. The enzymatic decomposition is typically conducted by incubating the collagen in an acidic environment (for example a pH of 1.0 to 2.0) at a temperature within a range from 30°C to 60°C, and preferably from 40°C to 50°C, for a period of 10 to 60 minutes, and preferably 15 to 40 minutes.

The enzymatic decomposition product obtained as a result of the above enzymatic decomposition is subjected to a reverse osmosis membrane treatment and recovered as a concentrated liquid. For the reverse osmosis membrane, a membrane having a salt blocking ratio of 10% to 50% is preferred, and examples of commercially available membranes include the products NTR-7410, NTR-7430 and NTR-7450 (all manufactured by Nitto Denko Corporation). During concentrating of the liquid, water is preferably added in an amount equivalent to 1 to 10 times the original liquid volume while the liquid is passed through the membrane.

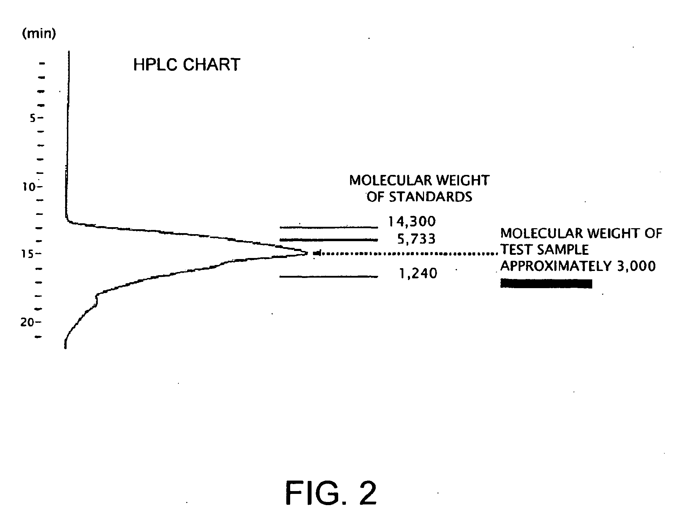

FIG. 2 shows the result of measuring the weight average molecular weight of the fish skin-derived low molecular weight collagen contained within the vascular aging inhibitor of the present embodiment. The measurement conditions involved using an HPLC analysis method under the conditions described below.
Column: TSK-gel guard column PWXL, TSK-gel G3000 PWXL, TSK-gel G2500 PWXL (all manufactured by Tosoh Corporation)
Mobile phase: 0.5% (w/v) NaCl aqueous solution
Flow rate: 0.8 ml/min.
Temperature: room temperature (23°C)
Detector: ultraviolet spectrometric detector (220 nm)
Sample: injection of 10 µl of a 1 to 2% (w/v) aqueous solution of fish skin-derived collagen
Standard sample: injection of 10 µl of an aqueous solution containing 1% (w/v) of each of three standards, namely lysozyme (derived from chicken egg white, weight average molecular weight: 14,300), insulin (derived from bovine pancreas, weight average molecular weight: 5,733), and bradykinin, weight average molecular weight: 1,240) (all manufactured by Sigma-Aldrich Co.).

Furthermore, a formulation according to the present embodiment is a formulation that contains the medicine described above in a granular form. Compared with capsules or tablets, producing the formulation in granular form accelerates the enzymatic decomposition that occurs when the formulation reaches the intestine.

### EXAMPLES

A description of the medicine and the formulation of the present invention using a working example is presented below. It should be noted that the scope of the present invention is in no way limited by the example described below.

### [Preparation of granular formulation]

Using the skin from gadiformes or pleuronectiformes as the raw material for the fish skin-derived collagen, and using pepsin as the decomposition enzyme, the pH was adjusted to 1.5, and an enzymatic decomposition was then conducted for 20 minutes at a temperature of 40°C. Subsequent measurement of the product under the conditions described above for the HPLC analysis method revealed a weight average molecular weight for the obtained fish skin-derived collagen of 3,000.

### [Test Example]

The granular formulation obtained from the above preparation was administered orally once per day to 12 test subj ects, in an amount sufficient to provide 5.0 g of the fish skin-derived collagen per administration. The state of the blood vessels in each of the 12 test subjects (labeled "test subject 1" to "test subject 12" in Table 1) was analyzed prior to commencing administration, and then after 3 months of administration, by using an ultrasound apparatus (LOGIQ 7 PRO, manufactured by GE Co.) to measure the thickness of the inner membrane of the carotid artery.

**[Table 1]**

| | Age (years) | Gender | Prior to administration | | | | After 3 months of administration | | | |
|---|---|---|---|---|---|---|---|---|---|---|
| | | | Carotid artery inner right side membrane thickness (mm) | Carotid artery inner left side membrane thickness (mm) | TC (mg/dl) | LDL-C (mg/dl) | Carotid artery inner right side membrane thickness (mm) | Carotid artery inner left side membrane thickness (mm) | TC (mg/dl) | LDL-C (mg/dl) |
| Test subject 1 | 71 | F | 0.75 | 0.87 | 280 | 241 | 0.5 | 0.7 | 263 | 172 |
| Test subject 2 | 50 | M | 0.66 | 0.75 | 228 | 158 | 0.51 | 0.62 | 235 | 145 |
| Test subject 3 | 67 | M | 0.89 | 1.04 | 277 | 240 | 0.85 | 0.76 | 209 | 118 |
| Test subject 4 | 50 | M | 0.92 | 1 | 258 | 170 | 1.03 | 1.03 | 302 | 218 |
| Test subject 5 | 58 | M | 0.7 | 0.7 | 148 | 53 | 0.73 | 0.65 | 157 | 67 |
| Test subject 6 | 61 | M | 0.7 | 1.45 | 317 | 227 | 0.62 | 1.35 | 282 | 199 |
| Test subject 7 | 66 | F | 0.8 | 1.3 | 252 | 138 | 0.75 | 1.03 | 277 | 167 |
| Test subject 8 | 48 | M | 0.73 | 0.95 | 272 | 177 | 0.62 | 0.75 | 270 | 170 |
| Test subject 9 | 56 | M | 0.7 | 1.55 | 297 | 218 | 0.62 | 1.25 | 283 | 208 |
| Test subject 10 | 71 | M | 1.3 | 1.59 | 205 | 130 | 1.17 | 1.44 | 197 | 127 |
| Test subject 11 | 59 | M | 0.7 | 1.1 | 191 | 118 | 0.7 | 1.03 | 195 | 127 |
| Test subject 12 | 64 | M | 1.37 | 1.24 | 245 | 155 | 1.24 | 0.82 | 248 | 167 |

| | | | | | | | | | | |
|---|---|---|---|---|---|---|---|---|---|---|
| Notes) TC: total cholesterol, LDL-C: low molecular weight cholesterol (commonly called "bad cholesterol"), inner membrane thickness values are measured values from the right and left sides of an ultrasound echo image. | | | | | | | | | | |

Arterial sclerosis is a phenomenon in which the inner membrane of the artery thickens, and if the innermembrane thickness exceeds 1 mm, then arterial sclerosis is diagnosed regardless of age. Currently, it is considered that the most accurate method for diagnosing arterial sclerosis involves measurement of the thickness of the inner membrane of the carotid artery using a precise ultrasound apparatus.

The 12 test subjects included 10 men and 2 women, with ages from 48 to 71 years, and an average age of 60.1 years. Those patients who were taking medication for hyperlipidemia stopped taking their medication one month prior to beginning the test, and were administered only the formulation of the present invention during the test period. As is evident from Table 1, as a result of the oral administration of the formulation prepared in the example over a 3 month period, 10 of the 12 test subjects exhibited a clear reduction in the thickness of the carotid artery inner membrane, and the thickness of fibrous plaque also decreased. Furthermore, a reduction in total cholesterol was confirmed in 4 of the 12 test subjects, and a reduction in LDL cholesterol was confirmed for 6 of the test subjects.

The foregoing description of the exemplary embodiments of the present invention has been provided for the purposes of illustration and description. The exemplary embodiments were chosen and described in order to best explain the principles of the invention and its practical applications, thereby enabling others skilled in the art to understand the invention for various embodiments and with the various modifications as are suited to the particular use contemplated.

## Claims

1. A medicine for use in reducing or preventing atherosclerosis, comprising a low molecular weight collagen derived from fish skin as an essential component, wherein a weight average molecular weight of the low molecular weight collagen derived from fish skin is approximately 3,000.

2. The medicine for use according to claim 1, wherein the low molecular weight collagen derived from fish skin is obtained by enzymatic decomposition using skin of gadiformes or pleuronectiformes as a raw material.

3. The medicine for use according to claim 1, for reducing the thickness of the inner membrane of the blood vessel.

4. A formulation for use in reducing or preventing atherosclerosis, comprising the medicine according to claim 1 in a granular form.

5. A formulation for use in reducing or preventing atherosclerosis, comprising the medicine according to claim 2 in a granular form.

6. Use of a low molecular weight collagen derived from fish skin in the preparation of a medicine for reducing or preventing atherosclerosis, wherein a weight average molecular weight of the low molecular weight collagen derived from fish skin is approximately 3,000.

7. Use according to claim 6, wherein the low molecular weight collagen derived from fish skin is obtained by enzymatic decomposition using skin of gadiformes or pleuronectiformes as a raw material.

8. Use according to claim 6, wherein the medicine is for reducing the thickness of the inner membrane of the blood vessel.

9. Use according to claim 6 or 7, wherein the medicine is in granular form.

## Patentansprüche

1. Arzneimittel zur Verwendung bei der Verringerung oder Prävention von Atherosklerose, umfassend ein von Fischhaut abgeleitetes Kollagen mit niedrigem Molekulargewicht als wesentliche Komponente, wobei ein gewichtsgemitteltes Molekulargewicht des von Fischhaut abgeleiteten Kollagens mit niedrigem Molekulargewicht ungefähr 3.000 beträgt.

2. Arzneimittel zur Verwendung nach Anspruch 1, wobei das von Fischhaut abgeleitete Kollagen mit niedrigem Molekulargewicht durch enzymatischen Abbau unter Verwendung von Haut von Gadiformes oder Pleuronectiformes als Rohmaterial erhalten wird.

3. Arzneimittel zur Verwendung nach Anspruch 1 zur Verminderung der Dicke der inneren Blutgefäßmembran.

4. Formulierung zur Verwendung bei der Verringerung oder Prävention von Atherosklerose, umfassend das Arzneimittel nach Anspruch 1 in Granulatform.

5. Formulierung zur Verwendung bei der Verringerung oder Prävention von Atherosklerose, umfassend das Arzneimittel nach Anspruch 2 in Granulatform.

6. Verwendung eines von Fischhaut abgeleiteten Kollagens mit niedrigem Molekulargewicht bei der Herstellung eines Arzneimittels zur Verringerung oder Prävention von Atherosklerose, wobei ein gewichtsgemitteltes Molekulargewicht des von Fischhaut abgeleiteten Kollagens mit niedrigem Molekulargewicht ungefähr 3.000 beträgt.

7. Verwendung nach Anspruch 6, wobei das von Fischhaut abgeleitete Kollagen mit niedrigem Molekulargewicht durch enzymatischen Abbau unter Verwendung von Haut von Gadiformes oder Pleuronectiformes als Rohmaterial erhalten wird.

8. Verwendung nach Anspruch 6, wobei das Arzneimittel zur Verminderung der Dicke der inneren Blutgefäßmembran vorgesehen ist.

9. Verwendung nach Anspruch 6 oder 7, wobei das Arzneimittel in Granulatform vorgesehen ist.

## Revendications

1. Médicament pour utilisation dans la réduction ou la prévention de l'athérosclérose, comprenant un collagène de faible masse moléculaire dérivé de peau de poisson en tant que composant essentiel, dans lequel la masse moléculaire moyenne en masse du collagène de faible masse moléculaire dérivé de peau de poisson est d'environ 3000.

2. Médicament pour utilisation selon la revendication 1, dans lequel le collagène de faible masse moléculaire dérivé de peau de poisson est obtenu par décomposition enzymatique utilisant la peau de gadiformes ou de pleuronectiformes en tant que matière première.

3. Médicament pour utilisation selon la revendication 1, pour réduire l'épaisseur de la membrane intérieure d'un vaisseau sanguin.

4. Formulation pour utilisation dans la réduction ou la prévention de l'athérosclérose, comprenant le médicament selon la revendication 1 sous forme granulaire.

5. Formulation pour utilisation dans la réduction ou la prévention de l'athérosclérose, comprenant le médicament selon la revendication 2 sous forme granulaire.

6. Utilisation d'un collagène de faible masse moléculaire dérivé de peau de poisson dans la préparation d'un médicament pour réduire ou prévenir l'athérosclérose, dans laquelle la masse moléculaire moyenne en masse du collagène de faible masse moléculaire dérivé de peau de poisson est d'environ 3000.

7. Utilisation selon la revendication 6, dans laquelle le collagène de faible masse moléculaire dérivé de peau de poisson est obtenu par décomposition enzymatique utilisant la peau de gadiformes ou de pleuronectiformes en tant que matière première.

8. Utilisation selon la revendication 6, dans laquelle le médicament est destiné à réduire l'épaisseur de la membrane intérieure d'un vaisseau sanguin.

9. Utilisation selon la revendication 6 ou 7, dans laquelle le médicament est sous forme granulaire.
